# EUROPÄISCHE PATENTSCHRIFT

(11) **EP 0 013 665 B1**
(45) Veröffentlichungstag und Bekanntmachung des Hinweises auf die Patenterteilung: **29.06.1983**
(21) Anmeldenummer: 80810008.5
(22) Anmeldetag: 09.01.1980
(51) Int. Cl.: C08G 73/06, C08G 73/02

(54) **Polymere Polyamin-1,3,5-triazine mit mindestens einem Polyalkylpiperidinrest, ihre Herstellung, ihre Verwendung zum Stabilisieren von organischem Material und das damit stabilisierte Material**
Polymeric polyamino 1,3,5-triazines containing at least one polyalkylpiperidine group, their preparation, their use in stabilizing an organic material and the material stabilized therewith
Polyamino-1,3,5-triazines polymères contenant au moins un reste polyalkylpipéridine, leur préparation, leur utilisation pour la stabilisation de matériaux organiques et le matériau ainsi stabilisé

(30) Priorität: 15.01.1979 CH 357/79
(43) Veröffentlichungstag der Anmeldung: 23.07.1980
(60) Teilanmeldung: 82104017.7
(73) Patentinhaber: CIBA-GEIGY AG, 4002 Basel (CH)
(72) Erfinder: Nikles, Erwin, Dr., CH-4410 Liestal (CH)

## Beschreibung

Die vorliegende Erfindung betrifft neue oligo- bzw. polymere Polyamino-1,3,5-triazine enthaltend mindestens einen Polyalkylpiperidinrest, ihre Herstellung und ihre Verwendung zum Stabilisieren von organischem Material.

Aus der DE-OS 2 636 144 sind bereits polymere Diamin-1,3,5-triazine enthaltend Polyalkylpiperidinreste zum Stabilisieren von organischem Material beschrieben. Es hat sich aber gezeigt, dass bei der Herstellung dieser Produkte zum Teil erhebliche Schwierigkeiten entstehen, da sich unlösliche, technisch schwer abtrennbare Nebenprodukte bilden.

Es ist nun gefunden worden, dass man ohne die erwähnten Schwierigkeiten polymere Polyamin-1,3,5-triazine erhalten kann, die eine ausgezeichnete stabilisierende Wirkung zeigen.

Demnach betrifft die vorliegende Erfindung neue Polyamino-1,3,5-triazine, erhältlich durch Reaktion eines Haloqen-1,3,5-triazines der Formel 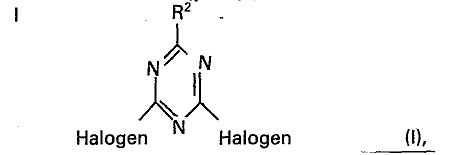 worin R² Halogen, Cyano, Azido, Hydrazido, Phenyl, ―OR⁷, ―SR⁷ oder―NR⁸R⁸' ist, wobei R⁷H, C₁―C₁₈ Alkyl, C₃―C₁₂ Alkenyl, C₃―C₁₈ Alkoxyalkyl, C₃―C₁₂ Cycloalkyl, C₇―C₁₈ Aralkyl, C₆―C₁₀ Aryl oder ein Rest der Formel II 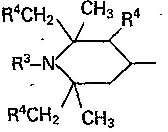 ist, worin R³ H, C₁―C₁₈ Alkyl, C₃―C₁₂ Alkenyl, C₃-C₅ Alkinyl, C₃―C₁₈ Alkoxyalkyl, gegebenenfalls durch Phenyl oder Phenoxy substituiertes C₂-C₄ Hydroxyalkyl oder C₇―C₁₈ Aralkyl und R⁴ H oder Methyl sind, und R⁸ und R⁸' unabhängig voneinander, H, C₁―C₂₃ Alkyl, das durch -0- unterbrochen sein kann, C₃―C₁₈ Alkenyl, C₃―C₅ Alkinyl, C₂―C₁₀ Hydroxyalkyl, C₂―C₅ Cyanalkyl, C₃―C₁₂ Cycloalkyl, C₇―C₁₈ Aralkyl, C₆―C₁₀ Aryl oder den Rest der Formel II bedeuten oder R⁸ und R^{8'} zusammen mit dem gemeinsamen N einen Pyrrolidin- oder einen gegebenenfalls durch C₁―C₄ Alkyl substituierten Piperidin-, Morpholin- oder Hexamethyleniminring bilden, mit einem Polyamin der Formel 111 oder einem Gemisch von mindestens 2 Polyaminen der Formel III 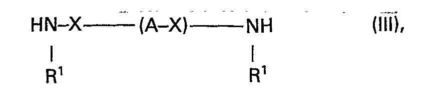 worin X C₂―C₆ Alkylen ist, A -O-, -S- oder―NR¹― sein kann, die Reste R¹ unabhängig voneinander H, C₁-C₂₃ Alkyl, das durch -0- unterbrochen sein kann, C₃―C₁₂ Cycloalkyl, C₇―C₁₈ Aralkyl, C₆―C₁₀ Aryl oder den Rest der Formel II bedeuten und c eine Zahl von 1 bis 4 ist, und gegebenenfalls Nachbehandlung des erhaltenen Produktes mit einem Acylierungsmittel und/oder mit einer Verbindung der Formel H―R^{2'}, worin R^{2'} die gleiche Bedeutung wie R² ausser Halogen und Phenyl hat, mit der Massgabe, dass mindestens einer der Reste R¹ oder R² eine Gruppe der Formel II ist oder enthält und dass im Falle der Umsetzung mit einem Gemisch von mindestens 2 Polyaminen der Formel III darin ein Amin der Formel III enthalten sein kann, worin c Null ist.

Bedeuten etwaige Substituenten C₁-C₁₈ Alkyl, so kann es sich um Methyl, Aethyl, n-Propyl, lsopropyl, n-Butyl, sec.-Butyl, n-Pentyl, n-Hexyl, n-Octyl, 1,1,3,3-Tetramethylbutyl, n-Nonyl, Decyl, Dodecyl, Tetradecyl, Hexadecyl oder Octadecyl handeln. Bevorzugt sind Alkylgruppen mit 1 bis 8 Kohlenstoffatomen und insbesondere solche mit 1 bis 4 Kohlenstoffatomen.

Stellen etwaige Substituenten C₁-C₂₃ Alkyl dar, so haben sie die gleiche Bedeutung wie die oben genannten und dazu noch beispielsweise Nonadecyl, Eicosyl, Heneicosyl, Docosyl und Tricosyl, wobei es sich immer um geradkettige oder verzweigte Kohlenwasserstoffe handeln kann.

Bedeuten etwaige Substituenten C₁―C₄ Alkyl, so handelt es sich um Methyl, Aethyl, n-Propyl, Isopropyl, n-Butyl, sek.-Butyl und tert.-Butyl.

Stellen etwaige Substituenten C₃―C₁₂ Cycloalkyl dar, so bedeuten sie beispielsweise Cyclopropyl, Cyclopentyl, Cyclohexyl, Cycloheptyl, Cyclooctyl, Cyclononyl oder Cyclododecyl. Bevorzugt ist Cyclohexyl.

Bedeuten etwaige Substituenten C₇―C₁₈ Aralkyl, so stellen sie insbesondere 1-Phenyläthyl, 1,1-Dimethylbenzyl, 2-Phenyläthyl oder vor allem Benzyl dar.

Stellen etwaige Substituenten C₆―C₁₀ Aryl dar, so bedeuten sie z. B. gegebenenfalls durch Methyl, Aethyl, n-Propyl, Isopropyl, n-Butyl, sec.-Butyl oder tert.-Butyl substituiertes Phenyl. Bevorzugt werden unsubstituierte Phenylgruppen.

Bedeuten etwaige Substituenten C₃―C₁₈ Alkenyl, so stellen sie insbesondere C₃―C₁₂ Alkenyl, wie beispielsweise Allyl, Methallyl, 2-Butenyl, 2-Hexenyl, 3-Hexenyl, 2-Octenyl, 2-Dodecenyl dar. Bevorzugt ist Allyl.

Bedeuten etwaige Substituenten C₃―C₅ Alkinyl, so handelt es sich z. B. um Propargyl, 1-Butinyl, 2-Butinyl oder n-1-Pentinyl. Bevorzugt ist Propargyl.

Stellen etwaige Substituenten C₃-C₁₈ Alkoxyalkyl dar, so kann der Alkylteil 1 bis 3 Kohlenstoffatome enthalten und der Alkoxyteil aus 1 bis 16 Kohlenstoffatomen bestehen, wie z.B. Aethoxymethyl, 2-Methoxyäthyl, 2-Aethoxyäthyl, 2-n-Butoxyäthyl, 3-n-Butoxyäthyl, 3-n-Butoxyäthyl, 2-Octoxyäthyl oder 2-Hexadecyloxyäthyl. Bevorzugt sind Alkoxyalkylgruppen mit 3 oder 4 Kohlenstoffatomen, z.B. Aethoxymethyl, 2-Methoxyäthyl oder 2-Aethoxyäthyl.

Bedeuten etwaige Substituenten C₂―C_{1O} Hydroxyalkyl, so stellen sie insbesondere C₂-C₄ Hydroxyalkyl dar, wie beispielsweise 2-Hydroxy-n-butyl, 2-Hydroxy-n-propyl und insbesondere 2-Hydroxyäthyl.

Stellen etwaige Gruppen Halogen dar, so handelt es sich z. B. um Fluor oder insbesondere um Chlor.
R⁷ und R⁸ bedeuten als C₂-C₅ Cyanalkyl insbesondere Cyanäthyl.
X stellt als C₂-C₆ Alkylen z. B. Aethylen, Propylen, Tetramethylen, Pentametylen und Hexamethylen dar. Bevorzugt sind Aethylen und Propylen.

Halogen ist bevorzugt Chlor.

Wird am Ende der Reaktion noch acyliert, so verfährt man nach an sich bekannten Methoden, wobei als Acylierungsmittel Carbonsäuren mit 1 bis 24 C-Atomen, bevorzugt 2 bis 8 C-Atomen oder deren Halogenide oder Anhydride verwendet werden, wie z.B. Essigsäure, Acetylchlorid, Essigsäureanhydrid, Propionsäureanhydrid, Butyrylchlorid oder Benzoylchlorid. Das nachträgliche Acylieren stellt eine bevorzugte Ausführungsform des Verfahrens dar.

Eine weitere bevorzugte Ausführungsform ist dadurch gekennzeichnet, dass man Cyanurchlorid mit mindestens einem Amin der Formel 111, worin c = 1 oder 2 ist, umsetzt und das Produkt nachfolgend mit einer Verbindung H―R^{2'} umsetzt, worin R^{2'} -OR⁷, ―SR⁷ oder -NR^{$}R⁸' mit der für R⁷, R⁸ und R^{8'} oben gegebenen Bedeutung ist, mit der Massgabe, dass mindestens einer der Reste R^{2'} oder R¹ eine Gruppe der Formel II mit der oben gegebenen Bedeutung ist oder enthält.

Eine weitere bevorzugte Ausführungsform des Verfahrens ist dadurch gekennzeichnet, dass man ein einheitliches Polyamin der Formel III verwendet, worin c die Zahl 1 oder 2 ist.

Wird ein Gemisch von Polyaminen der Formel III eingesetzt, in dem ein Amin der Formel 111, worin c gleich Null ist, enthalten ist, so überschreitet dessen Anteil vorzugsweise 50 Gew.-% des Polyamingemisches nicht.

Halogen-1,3,5-triazine der Formel 1, worin R² die oben angegebene Bedeutung ausser Halogen und Phenyl hat, können hergestellt werden, indem man ein Cyansäurehalogenid, bevorzugt -chlorid, mit 1 Mol einer Verbindung der Formel H-R² umsetzt.

Die Umsetzung der Halogentriazine mit den Verbindungen der Formel III oder den Verbindungen der Formel H-R² erfolgt entweder in wässriger Suspension oder in Gegenwart eines inerten Lösungsmittels, wie Aceton, Dioxan, Toluol, Xylol, in einem Temperaturbereich zwischen -10°C und der Siedetemperatur des Lösungsmittels. Die Reaktion erfolgt in Gegenwart organischer oder anorganischer Basen, um den Halogenwasserstoff zu binden.

Beispiele bevorzugter Basen sind Triäthylamin oder Tributylamin, Natriumhydroxyd, -carbonat oder -bicarbonat, Kaliumhydroxyd oder -carbonat oder Calciumcarbonat.

Polyamine der Formel 111, worin mindestens ein Rest R¹ eine Gruppe der Formel II bedeutet, erhält man nach an sich bekannten Methoden durch Umsetzen eines Polyamins der Formel IV 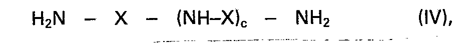 worin X und c die oben angegebene Bedeutung haben, mit einem Keton der Formel V 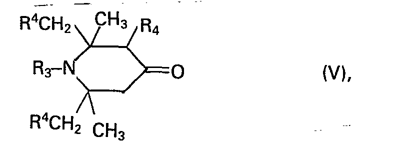 worin R³ und R⁴ die oben angegebene Bedeutung haben, mittels katalytischer Hydrierung.

Das Molverhältnis von Cyanursäurehalogeniden oder von Halogentriazinen der Formel I zu Polyaminen der Formel III ist abhängig vom Verhältnis der reaktiven Gruppen.

Eine bevorzugte Ausführungsform ist die zweistufige Umsetzung von Cyanurchlorid mit einem Polyamin der Formel III, worin mindestens ein Rest R¹ eine Gruppe der Formel II bedeutet, im Mol-Verhältnis von 0,8-1,2 : 1,2-0,8 unter Erhalt eines Chlors am Triazinring und die anschliessende Umsetzung mit einem Überschuss eines Amins der Formel HNR⁸R^{8'} .

Die erfindungsgemäss erhältlichen Polyaminotriazine können gemäss der vorliegenden Erfindung als Stabilisatoren für Kunststoffe gegen deren Schädigung durch Einwirkung von Sauerstoff, Wärme und Licht verwendet werden. Beispiele für solche Kunststoffe sind die in der DE-OS 2456864 auf den Seiten 12 bis 14 aufgeführten Polymeren.

Von besonderer Bedeutung ist die Stabilisierung von Polyolefinen, Styrolpolymerisaten und von Polyurethanen, für die sich die erfindungsgemässen Verbindungen hervorragend eignen. Beispiele hierfür sind Polyäthylen hoher und niedriger Dichte, Polypropylen, insbesondere auch in Form von Fasern, Bändchen und Folien, Aethylen-Propylen-Copolymerisate, Polystyrol, Styrol-Butadien-Acrylnitril-Copolymerisate, Mischungen von Polyolefinen oder von Styrolpolymerisaten, Polyurethane auf Polyäther- oder Polyesterbasis in Form von Filmen, Fasern, Lacken, Elastomeren oder Schaumstoffen. Besonders geeignet sind die erfindungsgemässen Verbindungen zum Stabilisieren von ABS.

Die Stabilisatoren werden den Kunststoffen in einer Konzentration von 0,01 bis 5 Gew.-%, berechnet auf das zu stabilisierende Material, zugesetzt. Vorzugsweise werden 0,03 bis 1,5, besonders bevorzugt 0,2 bis 0,6 Gew.-% der Verbindungen, berechnet auf das zu stabilisierende Material, in dieses eingearbeitet.

Die Einarbeitung kann nach der Polymerisation erfolgen, beispielsweise durch Einmischen der Verbindungen und gegebenenfalls weiterer Additive in die Schmelze nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels.

Die neuen Verbindungen können auch in Form eines Masterbatches, der diese Verbindungen beispielsweise in einer Konzentration von 2,5 bis 25 Gew.-% enthält, den zu stabilisierenden Kunststoffen zugesetzt werden.

Im Falle von vernetztem Polyäthylen werden die Verbindungen vor der Vernetzung beigefügt.

Als weitere Additive, mit denen zusammen die erfindungsgemäss verwendbaren Stabilisatoren eingesetzt werden können, sind beispielsweise zu nennen:
Antioxydantien, wie einfache 2,6-Dialkylphenole, Derivate von alkylierten Hydrochinonen, hydroxylierte Thiodiphenyläther, Alkylidenbisphenole, O-, N- und S-Benzylverbindungen, hydroxybenzylierte Malonester, Hydroxybenzyl-Aromaten, s-Triazinverbindungen, Amide der β-(3,5-Di-tert.-butyl-4-hydroxyphenyl)-propionsäure, Ester der β-(3,5-Di-tert.-butyl-4-hydroxyphenyl)-propionsäure, Ester der ß-(5-tert.-Butyl-4-hydroxy-3-methylphenyl)-propionsäure, Ester der 3,5-Di-tert.-butyl-4-hydroxyphenylessigsäure, Acylaminophenole, Benzylphosphonate, Aminoarylderivate, UV-Absorber und Lichtschutzmittel, wie 2-(2' -Hydroxyphenyl)-benztriazole, 2,4-Bis-(2' Hydroxyphenyl)-6-alkyl-s-triazine, 2-Hydroxybenzophenone, 1,3-Bis-(2'-hydroxybenzoyl)-benzole, Ester von gegebenenfalls substituierten Benzoesäuren, Acrylate, des weiteren Nickelverbindungen, sterisch gehinderte Amine, Oxalsäurediamide, Metalldesaktivatoren, Phospite, peroxidzerstörende Verbindungen, Polyamidstabilisatoren, basische Co-Stabilisatoren, PVC-Stabilisatoren, Nukleierungsmittel oder sonstige Zusätze, wie z. B. Weichmacher, Gleitmittel, Emulgatoren, Füllstoffe, Russ, Asbest, Kaolin, Talk, Glasfasern, Pigmente, optische Aufheller, Flammschutzmittel, Antistatica.

Beispiele für weitere Additive, mit denen zusammen die erfindungsgemäss verwendbaren Stabilisatoren eingesetzt werden können, finden sich in DE-OS 2427853 auf Seiten 18-24.

Die Herstellung und Verwendung der erfindungsgemässen Verbindungen wird in den folgenden Beispielen näher beschrieben. Die Temperaturen sind in Celsius-Graden angeben.

### Beispiel 1

100 g Triacetonamin und 36,5 g Diäthylentriamin wurden in 1,4 I Methanol gelöst, mit 1,5 g konz. Schwefelsäure versetzt und in Gegenwart von 5 g 5%iger Platinkohle bei 20 bis 25° unter Normaldruck in einer Wasserstoffatmosphäre geschüttelt. Nach 4 Stunden war die Wasserstoffaufnahme beendet (15,4 1, 0°, 10 KPa).

Der Katalysator wurde abfiltriert, das Lösungsmittel abgedampft und der Rückstand im Hochvakuum destilliert. Siedepunkt 161°/0.08 mmHg. Ausbeute: 92 g 1,7-Bis-(2,2,6,6-tetramethyl-4-pi- peridyl)-1,4,7-triazaheptan als leicht gelbliche Flüssigkeit.

19,0 g 1,7-Bis-(2,2,6,6-tetramethyl-4-piperidyl)-1,4,7-triazaheptan wurden in 200 ml Toluol gelöst, bei 0° tropfenweise unter Rühren mit einer Lösung von 9,2 g Cyanurchlorid in 50 ml Toluol und dann mit 6,6 g fein pulverisiertem Natriumhydroxid versetzt. Die Mischung wurde 1 Stunde bei 0 bis 5°, und 1 Stunde bei 40° gerührt und dann 16 Stunden unter Rückfluss gekocht. Nach dem Erkalten wurden die unlöslichen Teile abfiltriert und die Toluollösung eingedampft. Der Rückstand (16 g) wurde durch Kristallisation aus 200 ml Hexan in zwei Fraktionen zerlegt: Kristallisat, Schmelzpunkt: >260°, mittleres Molekulargewicht Mₙ: 8100, Mutterlauge eingedampft, Schmelzpunkt: > 260°, Mₙ: 2640.

### Beispiel 2

Man verfährt wie im Beispiel 1 beschrieben, setzt jedoch Cyanurchlorid mit 1,7-Bis(2,2,6,6-tetramethyl-4-piperidyl)-1,4,7-triazaheptan in verschiedenen molaren Verhältnissen um:

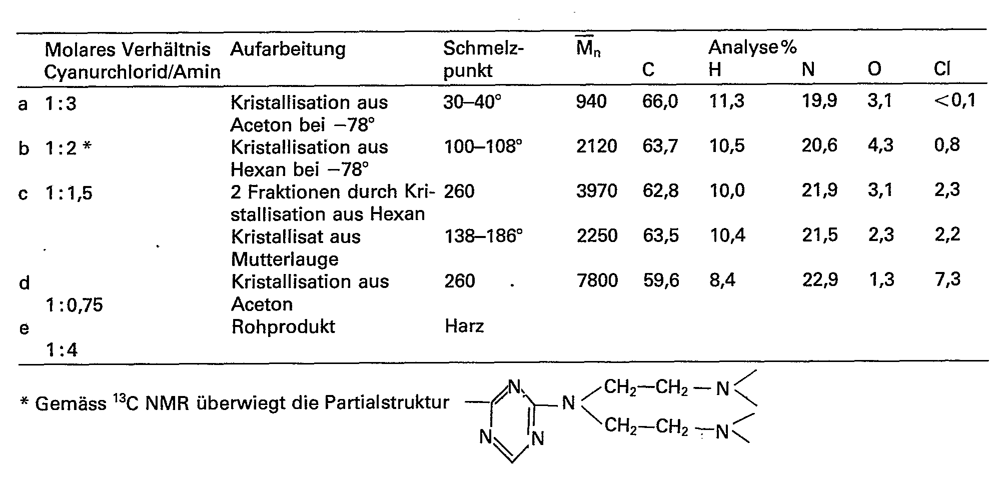

gegenüber

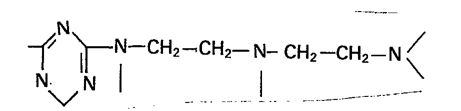

### Beispiel 3

Analog wie im Beispiel 1 beschrieben wurden 310 g Triacetonamin und 160 g Triäthylentetramin in 3 1 Methanol in Gegenwart von 2,5 g konz. Schwefelsäure und 15 g 5%ige Platinkohle hydriert. Das erhaltene 1,10-Bis(2,2,6,6-tetramethyl-4-piperidyl)-1,4,7,10-tetraazadecan siedet bei 182°/ 1,33 Pa.

21,7 g 1,10-Bis(2,2,6,6-tetramethyl-4-piperidyl)-1,4,7,10-tetraazadecan wurden in 200 ml Toluol gelöst und bei 0° unter Rühren mit einer Lösung von 9,1 g 2-Methylamino-4,6-dichlor-triazin in 100 ml Toluol versetzt. Nach Zugabe von 4,5 g fein pulverisiertem Natriumhydroxid wurde die Mischung 1 Stunde bei 0 bis 5°, 1 Stunde bei ca. 20° und 16 Stunden unter Rückfluss kochend gerührt. Die unlöslichen Teile wurden abfiltriert, das Filtrat eingedampft und der Rückstand aus 200 ml Cyclohexan kristallisiert. Schmelzpunkt: 130 bis 170°, Mₙ: 2700.

### Beispiel 4

Analog wie im Beispiel 3 beschrieben wurden die folgenden Dichlortriazine mit 1,7-Bis(2,2,6,6-tetramethyl-4-piperidyl)-1,4,7-triazaheptan im molaren Verhältnis 1 : 1 umgesetzt:

### Eingesetzte Dichlortriazinderivate

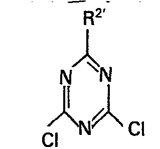

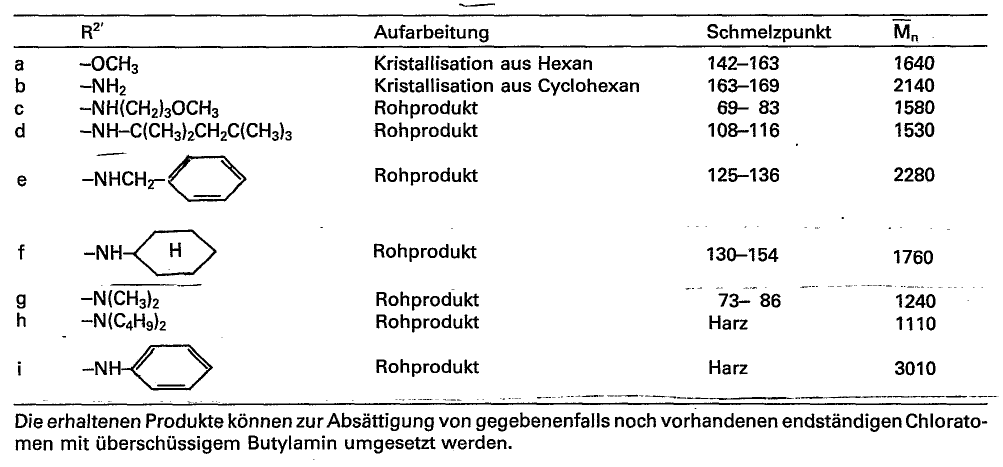

### Beispiel 5

Das wie im Beispiel 3 beschrieben erhaltene 1,10-Bis(2,2,6,6-tetramethyl-4-piperidyl)-1,4,7,10-tetraazadecan diente hier als Ausgangsprodukt.

25,6 1,10-Bis(2,2,6,6-tetramethyl-4-piperidyl)-1,4,7,10-tetraazadecan wurden in 200 ml Toluol gelöst und bei 0° portionenweise mit einer Lösung von 5,5 g Cyanurchlorid in 50 ml Toluol versetzt. Anschliessend wurden 4 g fein pulverisiertes Natriumhydroxid zugegeben. Die Mischung wurde 1 Stunde bei 0 bis 5°, 1 Stunde bei 40° und 16 Stunden unter Rückfluss kochend gerührt. Die unlöslichen Teile wurden abfiltriert, und die Lösung eingedampft. Der Rückstand wurde in Hexan aufgenommen, nochmals filtriert und das Produkt durch Kühlung mit Trockeneis ausgefällt. Schmelzpunkt 100 bis 120°, Mₙ: 2240.

### Beispiel 6

Analog wie im Beispiel 3 beschrieben, wurden Dichlortriazinderivate mit 1,10-Bis(2,2,6,6-tetramethyl-4-piperidyl)-1,4,7,10-tetraazadecan im molaren Verhältnis 1:1 umgesetzt. Die Rohprodukte wurden entweder umkristallisiert oder in einem Lösungsmittel aufgenommen, nochmals filtriert und eingedampft.

### Eingesetzte Dichlortriazinderivate

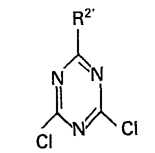

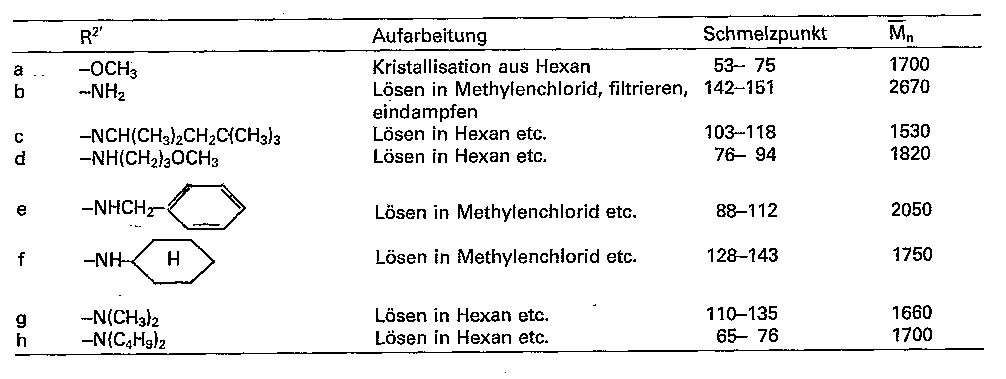

### Beispiel 7

Analog wie im Beispiel 1 beschrieben, wurde die Mischung von 155 g Triacetonamin, 113 g Diäthylentriamin, 2,5 g konz. Schwefelsäure in 2,5 I Methanol in Gegenwart von 7 g 5%iger Platinkohle hydriert. Die Destillation des Hydriergemisches ergab neben dem 1,7-Bis(2,2,6,6-tetramethyl-4-piperidyl)-1,4,7-triazaheptan bei 115 bis 118°/12 Pa das 1-(2,2,6,6-Tetramethyl-4-piperidyl)-1,4,7-triaza-heptan als Hauptprodukt.
a) 13,9 g 2-(1,1,3,3-Tetramethyl-1-butylamino)-4,6-dichlor-1,3,5-triazin wurden, analog wie im Beispiel 1 beschrieben, mit 12,1 g 1-(2,2,6,6-Tetramethyl-4-piperidyl)-1,4,7-triaza-heptan in Gegenwart von 5,4 g pulverisiertem Natriumhydroxid umgesetzt. Das Produkt wurde aus Hexan kristallisiert. Schmelzpunkt: 156 bis 164° C, Mₙ: 2020.
b) Bei der Umsetzung von 16,7 g 2-(1,1,3,3-Tetramethyl-l-butylamino)-4,6-dichlortriazin mit 9,7 g 1-(2,2,6,6-Tetramethyl-4-piperidyl)-1,4,7-tria- zaheptan (molares Verhältnis 3:2) in Gegenwart von 4,8 g pulverisiertem Natriumhydroxid wurde nach Kristallisation aus Hexan ein Produkt vom Smp. 160-196°C erhalten. M̅ₙ: 2380, Chlorgehalt 3,3%.

In analoger Weise wurden noch die folgenden Dichlortriazine mit dem 1-(2,2,6,6-Tetramethyl-4-piperidyl)-1,4,7-triazaheptan umgesetzt:

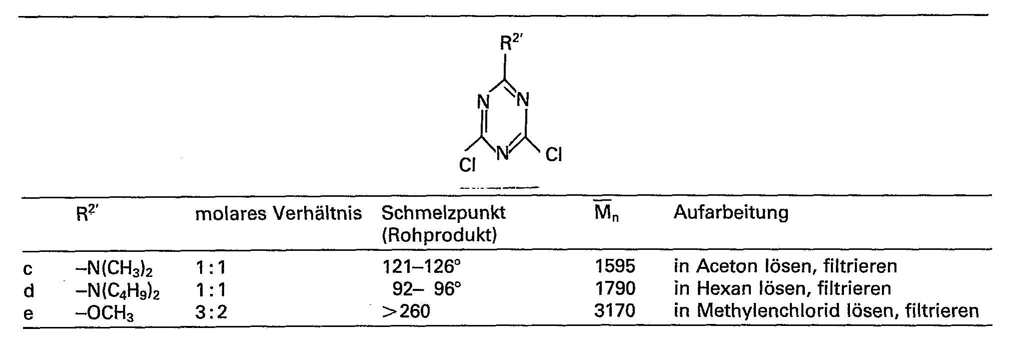

### Beispiel 8

Analog wie im Beispiel 1 beschrieben, wurden die folgenden Polyamine hergestellt:
a) 1,9-Bis(2,2,6,6-tetramethyl-4-piperidyl)-1,5,9-triazanonan (Siedepunkt 183°/66 Pa) aus Triacetonamin und Dipropylendiamin.
b) 1,9-Bis(2,2,6,6-tetramethyl-4-piperidyl)-5-methyl-1,5,9-triazanonan (Siedepunkt 200°/66 Pa) aus Triacetonamin und Bis-(3-aminopropyl)-methylamin.
c) 1,17-Bis(2,2,6,6-tetramethyl-4-piperidyl)-1,5,9,13,17-pentazaheptadecan (Siedepunkt 200°/ 0,13 Pa) aus Triacetonamin und Tetrapropylenpentamin.
d) 1,13-Bis(2,2,6,6-tetramethyl-4-piperidyl)-1,5,9,13-tetraazatridecan (Siedepunkt 176°/0,66 Pa) aus Triacetonamin und Tripropylentetramin.
e) 1,14-Bis(2,2,6,6-tetramethyl-4-piperidyl)-1,14-diaza-5,10-dioxatetradecan (Siedepunkt 173°/0,13 Pa) aus Triacetonamin und 4,9-Dioxatetradecan-1,12-diamin, in Gegenwart von 78 g 5%iger Platinkohle.
f) 1,9-Bis(2,2,6,6-tetramethyl-4-piperidyl)-5-cyclohexyl-1,5,9-triazanonan aus Triacetonamin und Bis(3-aminopropyl)-cyclohexylamin.
g) 1,9-Bis(2,2,6,6-tetramethyl-4-piperidyl)-5-n-butyl-1,5,9-triazanonan aus Triacetonamin und Bis(3-aminopropyl)butylamin.
h) 1,9-Bis(2,2,6,6-tetramethyl-4-piperidyl)-5-octadecyl-1,5,9-triazanonan aus Triacetonamin und Bis(3-aminopropyl)-octadecylamin.

i) 1,9-Bis(2,2,6,6-tetramethyl-4-piperidyl)-5-cyclododecyl-1,5,9-triazanonan aus Triacetonamin und Bis(3-aminopropyl)-cyclododecylamin.
k) 1,9-Bis(2,2,6,6-tetramethyl-4-piperidyl)-5-benzyl-1,5,9-triazanonan aus Triacetonamin und Bis(3-aminopropyl)-benzylamin.
I) 1,9-Bis(2,2,6,6-tetramethyl-4-piperidyl)-5-phenyl-1,5,9-triazanonan aus Triacetonamin und Bis(3-aminopropyl)-anilin.
m) 1,9-Bis(2,2,6,6-tetramethyl-4-piperidyl)-1,9-diaza-5-oxa-nonan (Sdp. 154°/66 Pa) aus Triacetonamin und Bis(3-aminopropyl)-äther.
n) 1,9-Bis(1,2,2,6,6-pentamethyl-4-piperidyl)-1,9-diaza-5-oxa-nonan aus Methyl-triacetonamin und Bis(3-aminopropyl)-äther.
o) 1-Isopropyl-7(2,2,6,6-tetramethyl-4-piperi- dyl)-1,4,7-triazaheptan (Sdp. 115°/66 Pa) aus 1-(2,2,6,6-Tetramethyl-4-piperidyl)-1,4,7-triazahep- tan und Aceton.
p) 1-Isobutyl-7(2,2,6,6-tetramethyl-4-piperidyl)-1,4,7-triazaheptan (Sdp. 115°/6,6 Pa) aus 1-(2,2,6,6-Tetramethyl-4-piperidyl)-1,4,7-triazaheptan und Isobutyraldehyd.
q) 1-Cyclohexyl-7(2,2,6,6-tetramethyl-4-piperi- dyl)-1,4,7-triazaheptan (Sdp. 151°/6,6 Pa) aus 1-(2,2,6,6-Tetramethyl-4-piperidyl)-1,4,7-triazahep- tan und Cyclohexanon.
r) Mischung von 1-(1,2,2,6,6-Pentamethyl-4-pi- peridyl)-1,4,7-triazaheptan und 1,7-Bis(1,2,2,6,6-Pentamethyl-4-piperidyl)-1,4,7-triazaheptan (Sdp. 143-162°/26 Pa) aus 1,2,2,6,6-Pentamethyl-4-pipe- ridon und Diäthylentriamin.
s) 1,7-Bis(2,6-diäthyl-2,3,6-trimethyl-4-piperi- dyl)-1,4,7-triazaheptan (Sdp. 160°/2,6 Pa) nach mehrfacher Destillation in einer Molekulardestillationsapparatur) aus 2,3,6-Trimethyl-2,6-diäthyl-4-piperidon und Diäthylentriamin in Gegenwart von 5% (bezogen auf die Edukte) 5%ige Platinkohle.
t) 1-Benzyl-7(2,2,6,6-tetramethyl-4-piperidyl)-1,4,7-triazaheptan (Sdp. 172°/52 Pa) aus 1-(2,2,6,6-Tetramethyl-4-piperidyl)-1,4,7-triazaheptan und Benzaldehyd.

Die obigen Polyamine können analog wie in den Beispielen 1, oder 9 beschrieben mit Cyanursäurederivaten zu oligomeren und polymeren Verbindungen umgesetzt werden.

### Beispiel 9

a) Zu einer Lösung von 95,4 g 1,7-Bis(2,2,6,6-tetramethyl-4-piperidyl)-1,4,7-triazaheptan in 2 I Toluol wurden unter Rühren bei 0° 46,1 g Cyanurchlorid in 1 I Toluol getropft. Anschliessend wurden bei derselben Temperatur 500 ml Natronlauge (In) portionenweise zugegeben. Die Mischung wurde zuerst 1 Stunde bei 0°, dann 1 Stunde bei Raumtemperatur und schliesslich 2½ Stunden bei 40° gerührt. Danach wurden durch Titration mit 0,1 n Salzsäure in 2 ml der wässrigen Phase noch 0,042 Milliäquivalente Base nachgewiesen.

Die wässrige Phase wurde abgetrennt, die toluolische Lösung zweimal mit Wasser gewaschen und bei max. 40° im Vakuum eingedampft. Der Rückstand wurde in Chloroform aufgenommen, klar filtriert und wieder schonend eingedampft. Man erhielt 100 g eines leicht gelblichen Produktes: Smp. < 260°, Mₙ: 3150, Chlorgehalt 7,23%. Das Polymere kann aus Hexan kristallisiert werden: farbloses Produkt, Smp. 256-281°, Mₙ: 4270, Chlorgehalt 6,98%.

In gleicher Weise wurde Cyanurchlorid mit Polyaminen im molaren Verhältnis 1:1 umgesetzt:

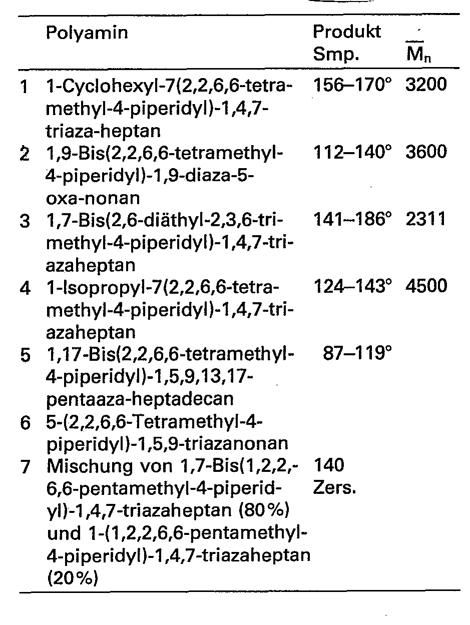

b) 10 g des aus Cyanurchlorid und 1,7-Bis(2,2,6,6-tetramethyl-4-piperidyl)-1,4,7-triaza- heptan hergestellten Rohproduktes wurden mit 30 ml n-Butylamin versetzt und 16 Stunden unter Rückfluss gekocht. Die entstandene Lösung wurde mit 100 ml Methylenchlorid verdünnt, dreimal mit 60 ml 10%iger Sodalösung gewaschen, überwasserfreiem Natriumsulfat getrocknet und eingedampft. Der Rückstand wurde mit Acetonitril behandelt. Das unlösliche Produkt wurde abfiltriert und im Vakuum getrocknet: 8,2 g, farblos, Smp. 156-158°C, Mₙ: 3800, Chlorgehalt 0,1 %, gut löslich in Methanol, Chloroform, Toluol, heiss löslich in Hexan, unlöslich in Acetonitril.

In genau derselben Weise können auch die folgenden Amine umgesetzt werden:

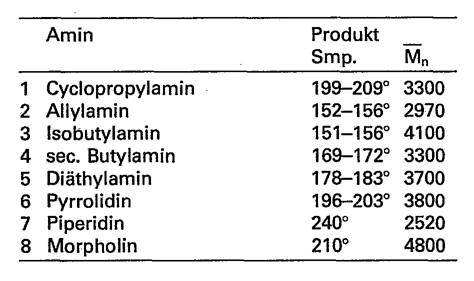

Ebenso wurden die folgenden aus Cyanurchlorid mit Polyaminen hergestellten Produkte mit Butylamin umgesetzt:

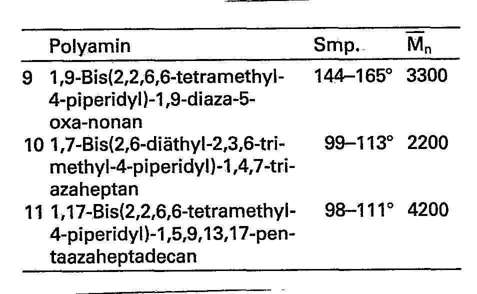

### Beispiel 10

6 g des gemäss Beispiel 9a) erhaltenen rohen Polymeren aus Cyanurchlorid und 1,7-Bis(2,2,6,6-tetramethyl-4-piperidyl)-1,4,7-triazaheptan wurden mit 20 ml 2-Aethyl-hexylamin 16 Stunden auf 130° erhitzt. Das Gemisch wurde wie in Beispiel 9b) für die Umsetzung mit n-Butylamin beschrieben, aufgearbeitet. Man erhielt 5,3 g eines farblosen Produktes vom Smp. 135-138°, Mₙ: 3600, das in Aceton, Chloroform, Toluol bei Raumtemperatur gut, in Hexan nur in der Wärme löslich ist.

Analog wurden die folgenden Amine umgesetzt:

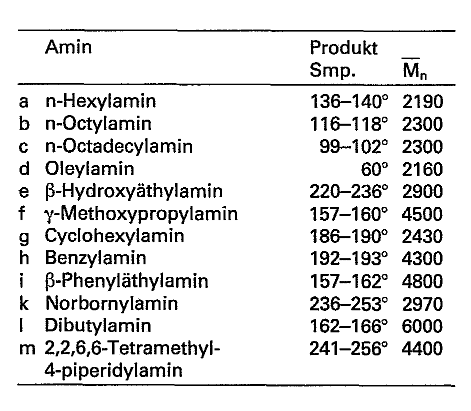

In gleicher Weise können auch umgesetzt werden: 3,5,5-Trimethylhexylamin, Isotridecylamin, 3-Isononyloxypropylamin, 3-Stearyloxypropylamin, 3,3,5-Trimethylcyclohexylamin, Cyclododecylamin.

### Beispiel 11

Analog wie in Beispiel 9a) beschrieben, wurde Cyanurchlorid mit 1,9-Bis(2,2,6,6-tetramethyl-4-pi- peridyl)-1,5,9-triazanonan und mit 1,10-Bis-(2,2,6,6-tetramethyl-4-piperidyl)-1,4,7,10-tetra- azadecan zu Polymeren umgesetzt, die pro Triazinring noch etwa 1-Chlor enthalten (Smp. 146-154°, Mₙ: 2640, bzw. Smp. 162-166°, Mₙ: 3270). Die weitere Reaktion mit den in der nachfolgenden Tabelle angegebenen n-Alkylaminen lieferte folgende Produkte:

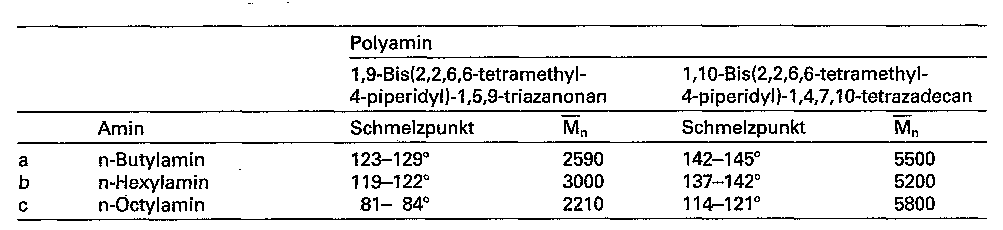

### Beispiel 12

96,4 g 1,14-Bis(2,2,6,6-tetramethyl-4-piperidyl)-1,14-diaza-5,10-dioxa-tetradecan, gelöst in 1 Toluol wurden bei 0° tropfenweise mit 36,8 g Cyanurchlorid in 200 ml Toluol versetzt. Anschliessend wurden portionenweise 400 ml I-n Natronlauge zugegeben und die Mischung zuerst 1 Stunde bei 0° und dann 4 Stunden bei 40° gerührt. Die Mischung wurde filtriert und die wässrige Phase abgetrennt. Die organische Phase wurde bei max. 90° im Vakuum eingedampft, in Methylenchlorid aufgenommen und nochmals filtriert. Nach dem Abdampfen des Lösungsmittels erhielt man das Produkt als weiches, klebriges Harz. Mₙ: 3045.

15 g des obigen Produktes wurden mit 50 ml n-Butylamin versetzt und 16 Stunden unter Rückfluss gekocht. Die Lösung wurde mit Methylenchlorid verdünnt, zweimal mit verdünnter Sodalösung und dann mit Wasser gewaschen, getrocknet und eingedampft. Als Rückstand wurde ein Produkt vom Smp. 66-92° C erhalten.

Mit n-Hexylamin wurde ein Produkt vom Smp. 60-76° erhalten.

### Beispiel 13

Analog wie in Beispiel 9) beschrieben, wurden 84,6 g 5-Methyl-1,9-Bis(2,2,6,6-tetramethyl-4-pipe- ridyl)-1,5,9-triazaheptan mit 36,8 g Cyanurchlorid umgesetzt, wobei ein Polymeres erhalten wurde, Mₙ: 2900, Smp. 140-176°.

Analog wie in den Beispielen 9) und 10) beschrieben, wurde das obige Polymere mit folgenden Aminen im Überschuss umgesetzt:

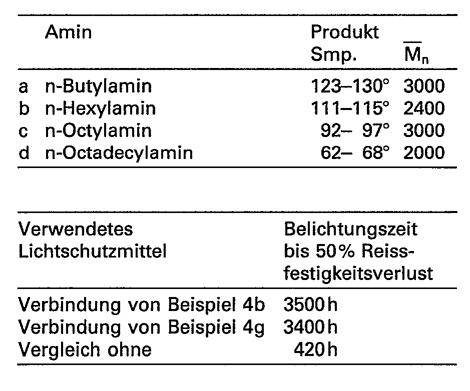

### Beispiel 14

50 g 2,4-Dichlor-6-phenyltriazin, 84,4 g 1,7-Bis(2,2,6,6-tetramethyl-4-piperidyl)-1,4,7-triaza- heptan, 17,7 g fein pulverisiertes Natriumhydroxyd und 500 ml Toluol wurden 16 Stunden unter Rückfluss gekocht. Die Mischung wurde klar filtriert und das Filtrat eingedampft. Der Rückstand schmilzt bei 136-151°.

### Beispiel 15

6,6 g des gemäss Beispiel 12 erhaltenen Produktes vom Smp. 60-76° wurden in 20 ml Methyläthylketon gelöst, mit 3,4 g fein pulverisiertem Kaliumcarbonat, 0,2 g Kaliumjodid und tropfenweise bei 80° mit 3,6 g Benzylbromid versetzt. Die Mischung wurde 40 Stunden unter Rückfluss gekocht, heiss mit 50 ml Methyläthylketon versetzt und filtriert. Das Filtrat wurde eingedampft. Der Rückstand wurde in Methylenchlorid gelöst, mit Wasser gewaschen, nach dem Abdampfen des Lösungsmittels mit Acetonitril bei -20° verrieben und nochmals filtriert. Man erhielt ein Benzylierungsprodukt vom Smp. 73-86°.

### Beispiel 16

a) 20 g des gemäss Beispiel 9a) hergestellten rohen Polymeren, 10 ml Aethylendiamin und 50 ml Toluol wurden 16 Stunden unter Rückfluss gekocht. Die Mischung wurde mit verdünnter Sodalösung und dann mit Wasser gewaschen. Die Lösung wurde über Natriumsulfat getrocknet und eingedampft. Der Rückstand wurde mit Acetonitril verrieben und filtriert. Man erhielt ein Produkt mit Smp. 203-206°, Mₙ: 4700.

Man verfuhr wie oben beschrieben, ersetzte jedoch das Aethylendiamin durch 20 ml Hydrazinhydrat oder 20 g Hexamethylendiamin und erhielt folgende Produkte: ^{_}

b) mit Hydrazinhydrat: Smp. 210°, Mₙ: 3800_

c) mit Hexamethylendiamin: Smp. 181°, Mₙ: 4200.

### Beispiel 17

14,8 g des gemäss Beispiel 9a) hergestellten rohen Polymeren wurde in 100 ml Toluol gelöst und mit 7 g Dodecylmercaptan, 35 mll-n Natronlauge und 65 ml Wasser versetzt. Die Mischung wurde während 24 Stunden bei Raumtemperatur gerührt, wobei in den ersten 8 Stunden Trimethylamingas im Überschuss eingeleitet wurde. Die Toluolphase wurde abgetrennt, mit Natronlauge und Wasser je zweimal gewaschen und eingedampft. Der Rückstand wurde bei -20° mit Acetonitril verrieben und abgetrennt. Man erhielt ein Produkt mit Smp. 66°, Mₙ: 3010.

### Beispiel 18

a) 14,8 g des gemäss Beispiel 9a) hergestellten Polymeren wurden in 100 ml n-Butanol suspendiert. Bei Raumtemperatur wurde während 6 Stunden Trimethylamingas durchgeleitet. Dann wurde das Gemisch 16 Stunden stehen gelassen. Anschliessend wurde eine Natrium-n-butanol-Lösung, hergestellt aus 1 g Natrium und 50 ml n-Butanol zugetropft. Nach 3stündigem Stehen bei Raumtemperatur wurde die Mischung eingedampft, in Methylenchlorid aufgenommen, mit Wasser gewaschen, über wasserfreiem Natriumsulfat getrocknet und eingedampft. Der Rückstand wurde mit Acetonitril angerieben, wobei ein Produkt vom Smp. 179-181° erhalten wurde, Mₙ: 5300.
b) Analog wurde mit obigem Ausgangsmaterial auch Cyclohexanolat in Cyclohexanol als Lösungsmittel umgesetzt. Smp. 194-225°, Mₙ: 3600.

### Beispiel 19

a) 12 g des gemäss Beispiel 9b) hergestellten Produktes wurden in 30 ml Toluol gelöst, mit 2,3 g Essigsäureanhydrid versetzt und 2 Tage bei Raumtemperatur stehen gelassen. Die Mischung wurde mit Toluol verdünnt, mit 2-n. Sodalösung gewaschen, getrocknet und eingedampft. Der Rückstand wurde mit Acetonitril behandelt, wobei 11 g eines farblosen Acetylierungsproduktes erhalten wurden. Smp. 196-207°, Mₙ: 4300.

Obiges Produkt enthält pro wiederkehrende Struktureinheit etwa eine Acetylgruppe.

b) Wurden 11,1 g Essigsäureanhydrid unverdünnt mit 12 g des obigen Ausgangsmaterials 14 Stunden gekocht, das überschüssige Anhydrid abdestilliert und der Rückstand wie oben beschrieben aufgearbeitet, so erhielt man ein Produkt vom Smp. 168-192°, das etwa 3 Acetylreste pro wiederkehrende Struktureinheit enthielt, Mₙ: 4000.

### Beispiel 20

a) Eine Lösung von 14,8 des gemäss Beispiel 9a) hergestellten Polymeren in 100 ml Toluol wurde mit Trimethylamingas gesättigt und 16 Stunden stehen gelassen. Anschliessend wurde unter Rühren bei Raumtemperatur eine Lösung von 1,95 g Kaliumcyanid in 5 ml Wasser zugetropft und während 3 Stunden ausreagieren gelassen. Die Toluol-Phase wurde abgetrennt, mit Wasser gewaschen und eingedampft. Der Rückstand wurde mit Acetonitril angerieben, filtriert und getrocknet. Das Produkt schmilzt bei 200-213°, Mₙ: 4300.
b) In der genau gleichen Weise wurden auch 2,15 g Natriumazid, gelöst in 5 ml Wasser umgesetzt. Das polymere Azid schmilzt bei 181-214°, Mₙ: 3800.

### Beispiel 21

a) 15 g des gemäss Beispiel 9b) hergestellten Polymeren wurden in 50 ml Toluol gelöst, bei 0-5° unter Rühren mit 6 g Benzoylchlorid und 85 ml I-n Natronlauge versetzt. Die Mischung wurde 14 Stunden bei 0°, dann 2 Tage bei Raumtemperatur gerührt. Die organische Phase wurde abgetrennt, mit 2-n Natronlauge und Wasser gewaschen, getrocknet, und eingedampft. Der Rückstand wurde mit Acetonitril verrieben und abfiltriert. Smp. 198-210°, Mₙ: 4300.

Das Produkt enthält pro wiederkehrende Struktureinheit etwa eine Benzoylgruppe.

b) Ebenso wurde das gemäss Beispiel 10) mit n-Hexylamin hergestellte Produkt benzoyliert. Smp. des erhaltenen Produktes 179-182°.

### Beispiel 22:

Die Mischung aus 12,1 g 2-Cyclohexylamino-4,6-dichlortriazin, 57 g 1,7-Bis(2,2,6,6-tetramethyl-4-piperidyl)-1,4,7-triazaheptan, 4,0 g fein pulverisiertem Natriumhydroxyd und 300 ml Toluol wurde 6 Stunden bei 40° gerührt und anschliessend 15 Stunden unter Rückfluss gekocht. Die unlöslichen Teile wurden abfiltriert und das Lösungsmittel abdestilliert. Das Produkt wurde in einer Kurzwegdestillierapparatur von nicht reagierten Ausgangsmaterialien befreit. Der Rückstand schmilzt bei 64-93°, Mn:1000.

### Beispiel 23

10 g des gemäss Beispiel 9a) hergestellten Polymeren wurden in 90 ml Chloroform gelöst und bei 0-5° unter Rühren mit 3,7 g Cyanurchlorid, gelöst in 10 ml Chloroform und mit 25 ml l-n Natronlauge versetzt. Die Mischung wurde bei 0° gerührt, bis 0,02 Mol Natronlauge verbraucht waren. Anschliessend wurden 5 g n-Butylamin zugegeben und die Mischung bei 40°, dann 60° während 14 Stunden reagieren gelassen. Die organische Phase wurde abgetrennt, mit 2-n Natronlauge und Wasser gewaschen, getrocknet und eingedampft. Das Produkt enthält pro Triazinring noch ein Chlor. Es kann wie im Beispiel 9b) beschrieben mit Aminen zu polymeren Triaminotriazinen umgesetzt werden.

### Beispiel 24

Lichtschutzwirkung in Polypropylen-Fasern (130/37)

1000 Teile unstabilisiertes Polypropylenpulver (Schmelzindex - 15) werden in einem Schnellmischer mit 0,5 Teilen Calcium (3,5-ditert.Butyl-4-hydroxy-benzyl-monoethylphosphonat),1 Teil Ca-Stearat und mit 3 Teilen der in der Tabelle angeführten Lichtschutzmittel gemischt und anschliessend in einem Extruder bei 220° extrudiert und granuliert. Das erhaltene Granulat wird in einer Labor-Schmelzspinnanlage bei einer maximalen Temperatur von 270° und einer Geschwindigkeit von 600 m/Minute zu einem 403/37 denier Multifilament versponnen. Dieses wird verstreckt und gezwirnt mittels einer Streckzwirnmaschine. Das Streckverhältnis ist 1:3,2, so dass schliesslich Multifilamente 130/37 denier erhalten werden. Die Multifilamente werden auf weissen Karton montiert und im Xenotest 1200 belichtet.

Als Mass der Schutzwirkung gilt die Belichtungszeit bis 50% Reissfestigkeitsverlust.

Die Ergebnisse sind in der Tabelle zusammengefasst.

## Patentansprüche

1. Polyaminotriazine, erhältlich durch Reaktion eines Halogen-1,3,5-triazines der Formel 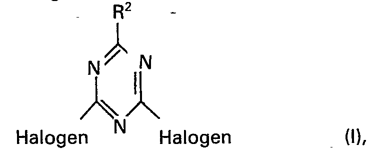 worin R^{L} Halogen, Cyano, Azido, Hydrazido, Phenyl,-OR⁷, ―SR⁷ oder―NR⁸R^{8'} ist, wobei R⁷ H, C₁―C₁₈ Alkyl, C₃―C₁₂ Alkenyl, C₃―C₁₈ Alkoxyalkyl, C₃―C₁₂ Cycloalkyl, C₇―C₁₈ Aralkyl, C₆―C₁₀ Aryl oder ein Rest der Formel II 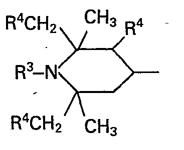 ist, worin R³ H, C₁―C₁₈ Alkyl, Cₐ-C₁₂ Alkenyl, C₃-C₅ Alkinyl, C₃―C₁₈ Alkoxyalkyl, gegebenenfalls durch Phenyl oder Phenoxy substituiertes C₂-C₄ Hydroxyalkyl oder C₇―C₁₈ Aralkyl und R⁴ H oder Methyl sind, und R⁸ und R⁸' unabhängig voneinander, H, C₁―C₂₃ Alkyl, das durch -O- unterbrochen sein kann, C₃―C₁₈ Alkenyl, C₃-C₅ Alkinyl, C₂―C₁₀ Hydroxyalkyl, C₂―C₅ Cyanalkyl, C₃―C₁₂ Cycloalkyl, C₇―C₁₈ Aralkyl, C₆―C₁₀ Aryl oder den Rest der Formel II bedeuten oder R⁸ und R^{8'} zusammen mit dem gemeinsamen N einen Pyrrolidin- oder einen gegebenenfalls durch Cₗ-C₄ Alkyl substituierten Piperidin-, Morpholin- oder Hexamethyleniminring bilden, mit einem Polyamin der Formel III oder einem Gemisch von mindestens 2 Polyaminen der Formel III 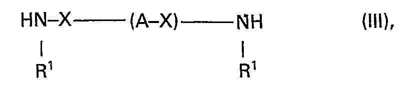 worin X C₂―C₆ Alkylen ist, A -O, -S- oder ―NR¹― sein kann, die Reste R¹ unabhängig voneinander H, C₁-C₂₃ Alkyl, das durch -O- unterbrochen sein kann, C₃―C₁₂ Cycloalkyl, C₇―C₁₈ Aralkyl, C₆―C₁₀ Aryl oder den Rest der Formel II bedeuten und c eine Zahl von 1 bis 4 ist, und gegebenenfalls Nachbehandlung des erhaltenen Produktes mit einem Acylierungsmittel und/oder mit einer Verbindung der Formel H―R^{2'}, worin R^{2'} die gleiche Bedeutung wie R² ausser Halogen und Phenyl hat, mit der Massgabe, dass mindestens einer der Reste R¹ oder R² eine Gruppe der Formel II ist oder enthält und dass im Falle der Umsetzung mit einem Gemisch von mindestens 2 Polyaminen der Formel III darin ein Amin der Formel III enthalten sein kann, worin c null ist.

2. Polyaminotriazine gemäss Anspruch 1, erhältlich durch Reaktion von Cyanursäurechlorid mit mindestens einem Amin der Formel III, worin c = 1 oder 2 ist, und nachfolgender Umsetzung mit einer Verbindung H-R²', worin R²' -OR7, -SR⁷ oder ―NR⁸R^{8'} mit der für R⁷, R⁸ und R^{8'} in Anspruch 1 gegebenen Bedeutung ist, mit der Massgabe, dass mindestens einer der Reste R^{2'} oder R¹ eine Gruppe der Formel II mit der in Anspruch 1 gegebenen Bedeutung ist oder erhält.

3. Polyaminotriazine gemäss Anspruch 2, erhältlich durch Reaktion von Cyanursäurechlorid mit III im Molverhältnis von 0,8-1,2:1,2-0,8.

4. Polyaminotriazine gemäss Anspruch 1 oder 2, erhältlich durch Reaktion von I mit III und Nachbehandlung mit einem Carbonsäureanhydrid oder Carbonsäurehalogenid als Acylierungsmittel.

5. Verwendung der Polyaminotriazine des Anspruchs 1 zum Stabilisieren von organischem Material, insbesondere von Kunststoffen gegen deren Schädigung durch Einwirkung von Sauerstoff, Wärme und Licht.

## Claims

1. A polyaminotriazine obtainable by reaction of a halo-1,3,5-triazine of the formula I 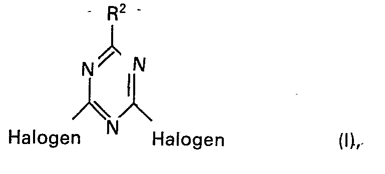 wherein R² is hydrogen, cyano, azido, hydrazino, phenyl, -OR⁷, -SR⁷ or -NR⁸R^{8'} , in which R⁷ is hydrogen, C₁―C₁₈― alkyl, C₃-C₁₂alkenyl, C₃―C₁₈alkoxyalkyl, C₃―C₁₂cycloalkyl, C₇―C₁₈aralkyl, C₆―C₁₀aryl or a radical of the formula II 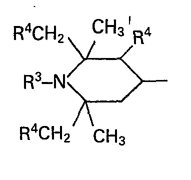 wherein R³ is hydrogen, C₁―C₁₈alkyl, C₃―C₁₂alkenyl, C₃―C₅alkynyl, C₃―C₁₈alkoxyalkyl, C₂-C₄hydroxyalkyl which is unsubstituted or substituted by phenyl or phenoxy, or is C₇―C₁₈aralkyl, and R⁴ is hydrogen or methyl, and R⁸ and R^{8'} , each independently of the other, can be hydrogen, C₁―C₂₃―alkyl which can be interrupted by oxygen, C₃―C₁₈alkenyl, C₃―C₅alkynyl, C₂―C₁₀hydroxyalkyl, C₂―C₅cyanoalkyl, C₃―C₁₂cycloalkyl, C₇―C₁₈aralkyl, C₆―C₁₀aryl or the radical of the formula II, or R⁸ and R^{8'} together with the nitrogen atom to which they are attached form a pyrrolidine ring or a piperidine, morpholine, or hexamethyleneimine ring, each of which is unsubstituted or substituted by C₁-C₄alkyl, with a polyamine of the formula III or with a mixture of at least 2 polyamines of the formula III 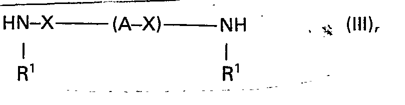 wherein X is C₂―C₆alkylene, A may be -0-, -S- or ―NR¹―, each R¹ independently of the other is hydrogen, Cₗ-C₂₃alkyl which can be interrupted by oxygen, C₃―C₁₂cycloalkyl, C₇―C₁₈―aralkyl, C₆―C₁₀aryl or the radical of the formula II, and c can be an integer from 1 to 4, and, if desired, subsequently acylating the reaction product with an acylating agent and/or with a compound of the formula H―R^{2'} , wherein R^{2'} has the same meaning as R² except halogen and phenyl, with the proviso that at least one or R¹ or R² is or contains a group of the formula II, and that, if the reaction is carried out with a mixture of at least two polyamines of the formula II, said mixture may contain an amine of the formula III, wherein c is O.

2.A polyaminotriazine according to claim I, obtainable by reaction of cyanuric chloride with at least one amine of the formula III, wherein c is 1 or 2, and subsequent reaction with a compound of. the formula H-R²', wherein R^{2'} is -OR⁷, -SR⁷ or -NR⁸R^{8'} , and R⁷, R⁸ and R^{8'} are as defined in claim 1, with the proviso that at least one of R²' or R¹ is or contains a group of the formula II as defined in claim 1.

3. A pol^{y}aminotriazine according to claim 2, obtainable by reaction of cyanuric chloride with the compound of formula III in the molar ratio of 0.8-1.2:1.2-0.8.

4. A polyaminotriazine according to either claim 1 or claim 2, obtainable by reaction of I with III and subsequent treatment with a carboxylic acid anhydride or carboxylic acid halide as acylating agent.

5. Use of a polyaminotriazine of claim 1 for stabilising organic material, in particular plastics, against damage by the action of oxygen, heat and light.

## Revendications

1. Polyamino-triazines que l'on peut obtenir en faisant réagir une halogéno-triazine-1,3,5 répondant à la formule I: 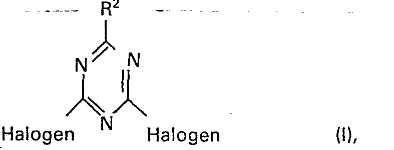 dans laquelle R² représente u'n halogène ou un radical cyano, azido, hydrazido, phényle, -OR⁷, -SR⁷ ou -NR⁸R^{8'}, où R⁷ représente H, un alyle en C₁-C₁₈, un alcényle en C₃-C₁₂, un alcoxyalkyle en C₃-C₁₈, un cycloalkyle en C₃-C₁₂, un aralkyle en C₇-C₁₈, un aryle en C₆-C₁₀ ou un radical répondant à la formule II 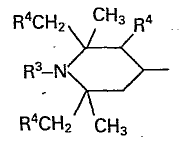 dans laquelle R³ représente H, un alkyle en C₁-C₁₈, un alcényle en C₃-C₁₂, un alcynyle en C₃-C₅, un alcoxyalkyle en C₃-C₁₈, un hydroxy-alkyle en C₂-C₄ éventuellement porteur d'un phényle ou d'un phé- noxy, ou un aralkyle en C₇-C₁₈, et R⁴ représente H ou un méthyle, et R⁸ et R⁸' représentent chacun, indépendamment l'un de l'autre, l'hydrogène, un alkyle en C₁-C₂₃ éventuellement interrompu par -O-, un alcényle en C₂-C₁₈, un alcynyle en C₃-C₅, un hydroxy-alkyle en C₂-C₁₀, un cyanalkyle en C₂-C₅, un cycloalkyle en C₃-C₁₂, un aralkyle en C₇-C₁₈, un aryle en C₆-C₁₀ ou un radical de formule II, ou encore R⁸ et R⁸' forment ensemble et avec l'atome d'azote commun un noyau de pyrrolidine ou un noyau de pipéridine, de morpholine ou de perhydro-azépine éventuellement porteur d'un alkyle en C₁-C₄, avec une polyamine de formule III ou un mélange d'au moins deux polyamines répondant à la formule III: 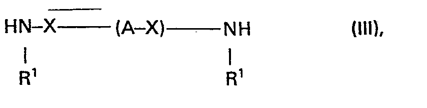 dans laquelle X représente un alkylène en C₂-C₆, A représente -O-, -S- ou NR¹-, les R¹ représentent chacun, indépendamment l'un de l'autre, l'hydrogène, un alkyle en C₁-C₂₃ éventuellement interrompu par -O-, un cycloalkyle en C₃-C₁₂, un aralkyle en C₇-C₁₈, un aryle en C₆-C₁₀ ou un radical de formule II, et c représente un nombre de 1 à 4, et éventuellement en traitant ensuite le produit obtenu par un agent d'acylation et/ou par composé de formule H-R^{2'} dans lequel R^{2'} a la même signification que R² mais ne peut représenter ni un halogène ni un phényle, avec la condition qu'au moins l'un des symboles R¹ et R² soit ou contienne un radical de formule II et que, dans le cas de la réaction avec un mélange d'au moins deux polyamines de formule III, il puisse y avoir dans ce mélange une amine de formule III dans laquelle c est égal à 0.

2. Polyaminotriazines selon la revendication 1 qui peuvent être obenues par réaction du chlorure de cyanuryle avec au moins une amine de formule III dans laquelle c est égal à 1 ou à 2, et réaction ultérieure avec un composé H-R^{2'} dans lequel R^{2'} représente un radical répondant à l'une des formules-OR⁷,-SR⁷ et-NR⁸R^{8'} dans lesquelles R⁷, R⁸ et R^{8'} ont les significations données dans la revendication 1, avec la condition qu'au moins l'un des radicaux R^{2'} et R¹ soit ou contienne un radical de formule II tel que défini à la revendication 1.

3. Polyaminotriazines selon la revendication 2 qui peuvent être obtenues par réaction du chlorure de cyanuryle avec un composé (III) dans un rapport molaire de 0,8-1,2:1,2-0,8.

4. Polyaminotriazines selon l'une des revendications 1 et 2, qui peuvent être obtenues par réaction de (I) avec (III) et traitement ultérieur avec un anhydride d'acide carboxylique ou un halogénure d'acide carboxylique comme agent d'acylation.

5. Application des polyaminotriazines selon la revendication 1 à la stabilisation de matières organiques, plus particulièrement de matières plastiques, contre leur dégradation par l'action de l'oxygène, de la chaleur et de la lumière.
